Europäisches Patentamt

European Patent Office

Office européen des brevets

(11) Publication number: **0 231 944 B1**

# EUROPEAN PATENT SPECIFICATION

(45) Date of publication of patent specification: **29.12.93**  (51) Int. Cl.⁵: **C07C 231/06**, C07C 233/09, B01J 23/72

(21) Application number: **87101559.0**

(22) Date of filing: **05.02.87**

The file contains technical information submitted after the application was filed and not included in this specification

(54) **Method for stopping and starting a acrylamide reactor.**

(30) Priority: **05.02.86 JP 22008/86**

(43) Date of publication of application:
**12.08.87 Bulletin 87/33**

(45) Publication of the grant of the patent:
**29.12.93 Bulletin 93/52**

(84) Designated Contracting States:
**AT DE ES FR GB IT NL**

(56) References cited:
EP-A- 78 178          DE-A- 2 001 903
DE-A- 2 504 640      GB-A- 1 392 380
GB-A- 1 393 976      US-A- 4 313 808

Japanese chemical society
(1981)p.1825-1827

(73) Proprietor: **MITSUI TOATSU CHEMICALS, Inc.**
**2-5 Kasumigaseki 3-chome**
**Chiyoda-Ku Tokyo 100(JP)**

(72) Inventor: **Asano, Shiro**
**4-7, Kamo**
**Takaishi-shi Osaka-fu(JP)**
Inventor: **Shizuka, Kohei**
**631-3, Andou**
**Fujisawa-shi Kanagawa-ken(JP)**
Inventor: **Kambara, Yoshihiko**
**6-3-213, Higashihagoromo**
**Takaishi-shi Osaka-fu(JP)**

(74) Representative: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

**Description**

Background of the Invention

a) Field of the Invention

This invention relates to improvements in a process for the synthesis of acrylamide by using a metallic copper-based catalyst as a suspended bed to react acrylonitrile with water in the liquid phase. More particularly, it relates to a method for stopping and starting the reactor when it is desired to discharge the catalyst from the reactor and thereby shut down the reactor.

b) Description of the Prior Art

As will be described later, the most common industrial process for the synthesis of acrylamide is one in which acrylonitrile is reacted with water in the liquid phase by using a metallic copper-based catalyst as a suspended bed. Since this reaction is usually carried out at an elevated temperature of 70 to 150°C, acrylonitrile, acrylamide and/or other impurities tend to polymerize, it has been known that such polymerization can be prevented by using various types of stabilizers which will be described later.

In processes using such a stabilizer, polymerization can be effectively controlled during ordinary operation of the reactor. Nevertheless, the present inventors' experience indicates that polymerization reactions often take place immediately after the beginning of operation and, at the same time, an abrupt deactivation of the catalyst is observed. In such a case, the progress of polymerization can be suppressed by adding a large amount of stabilizer. However, the expensive catalyst must be replaced because the activity of the catalyst once deactivated is not restored, and the large amount of stabilizer added to the reaction system must be removed from the resulting product. This involves great difficulties from technical and economical points of view. Accordingly, it would be desirable to establish a new method for the prevention of polymerization which may characteristically occur within the reactor immediately after the beginning of operation.

From GB-A-1 393 976 a process for the hydration of acrylonitrile with water in the presence of a free metallic copper catalyst has been known which is concerned with the removal of oxygen from the raw feed materials.

As a result of close investigation on the cause of polymerization immediately after the beginning of operation, the present inventors have found that such polymerization is associated with the small amount of catalyst remaining in the reactor after stopping its operation and discharging the catalyst. The present invention has been completed on the basis of this finding.

Summary of the Invention

It is an object of the present invention to provide a method for stopping and starting a reactor which can prevent a reduction in catalytic activity (more specifically, a rapid reduction in the degree of conversion of acrylonitrile) that may often be encountered in the synthesis of acrylamide by reacting acrylonitrile with water in the presence of a metallic copper-based catalyst used as a suspended bed and, in particular, immediately after the resumption of operation by using a previously stopped reactor.

It is another object of the present invention to provide a method for stopping and starting a reactor which can perfectly prevent the occurrence of polymerization phenomena within the reactor without contaminating the resulting product with a stabilizer.

It is still another object of the present invention to provide a method for stopping and starting a reactor in which, where an aqueous acrylamide solution containing no catalyst particles is obtained by passing the reacted solution through a catalyst filter to separate the fine catalyst particles present therein, it is possible to continue stable production while removing the very small amount of fine catalyst particles formed and while preventing the flow resistance of the filter from increasing abnormally.

In a process for the synthesis of acrylamide by reacting acrylonitrile with water in the presence of a metallic copper-based catalyst used as a suspended bed, the above objects of the present invention are accomplished by a method for stopping an acrylamide reactor by discharging the catalyst from the reactor after a continuous reaction and restarting said reactor which comprises discharging the catalyst, or discharging the catalyst and maintaining the reactor by step a) or b) mentioned below, in such a way that the amount of copper oxides present in the metallic copper-based catalyst remaining in the reactor, as expressed in terms of metallic copper, is not greater than 0.07% by weight based on the amount of metallic copper catalyst used at the beginning of the operation.

In detail the method of the invention is characterized in that the following steps are carried out:

a) discharging the catalyst by the procedure of washing or dissolving at least a part of the metallic copper and/or copper oxides and washing off the resulting solution, whereby the total amount of metallic copper and copper oxides remaining in the reactor is decreased to not greater than 0.07% by weight based on the amount of metallic copper catalyst used at the beginning of the operation, or

b) discharging the catalyst, and then the reactor is either filled with water or inert gas or evacuated so that the partial pressure of oxygen within the reactor is kept at $6.66 \cdot 10^3$ Pa (50 torr) or less, whereby the amount of copper oxides remaining in the reactor prior to restart is decreased to not greater than 0.07% by weight based on the amount of metallic copper catalyst used at the beginning of the operation.

In the present method a stirred reactor having at least a heat exchanger, a catalyst settler or a catalyst filter disposed on the inside or outside thereof is used.

Especially in the case of discharging the catalyst and then establishing an inert environment within the reactor, the fraction of metallic copper catalyst which is difficult to discharge may be allowed to remain in the reactor, so that the washing-off operation can be simplified.

Effective procedures for discharging the catalyst or for discharging the catalyst and maintaining the reactor include washing off the catalyst; discharging most of the catalyst and then dissolving out the residual catalyst; discharging most of the catalyst and then filling the reactor with water or an inert gas without allowing the interior of the reactor to come into contact with the atmosphere; discharging most of the catalyst and then evacuating the reactor for the same purpose, and thus keeping the partial pressure of oxygen within the reactor at $6.66 \cdot 10^3$ Pa (50 torr) or less by any of the foregoing means. In this manner, the amount of copper oxides present in the metallic copper-based catalyst remaining in the reactor can be controlled so as to permit safe shut-down of an acrylamide reactor.

Brief Description of the Drawings

Fig. 1 is a schematic illustration of a suspended bed reactor used in some of the Examples and Comparative Examples given later; and

Fig. 2 is a schematic illustration of another suspended bed reactor.

In these figures, a raw material feed pipe is shown at 1, a reacted solution discharge pipe at 2, a catalyst settler section at 3, a heating steam coil at 4, a stirrer at 5, a catalyst discharge valve at 6, and slanting plates at 7.

Description of the Preferred Embodiments

The metallic copper-based catalyst used in the present invention is a catalyst whose principal active component is considered to be metallic copper. Typical examples thereof include:

(A) Vapor phase reduced copper obtained by reducing copper oxide with hydrogen gas [as described in U.S. Patents 3,631,104 and 3,928,439 and in Shokubai, 27, No. 6, 365(1985)];

(B) Liquid phase reduced copper obtained by treating a copper salt or copper hydroxide with a reducing agent such as a borohydride in the liquid phase [as described in U.S. Patent 3,939,205 and Japanese Patent Laid-Open Nos. 23717/'73, 68519/'73, 19616/'77 and 65259/'83];

(C) Decomposition products of copper hydride obtained by reacting a copper salt or copper hydroxide with a hypophosphite in the liquid phase [as described in U.S. Patent 3,929,881];

(D) Decomposition products of similarly decomposable copper compounds [as described in Japanese Patent Laid-Open No. 26724/'73 and U.S. Patent 3,936,502]; and

(E) Raney copper obtained by treating a Raney copper alloy with a basic compound [as described in U.S. Patents 4,056,565, 3,920,740 and 3,928,440 and Japanese Patent Publication No. 22019/'75].

These catalysts are appropriately prepared in the form of a relatively fine powder and used in the suspended state by charging it into the suspended bed reactor.

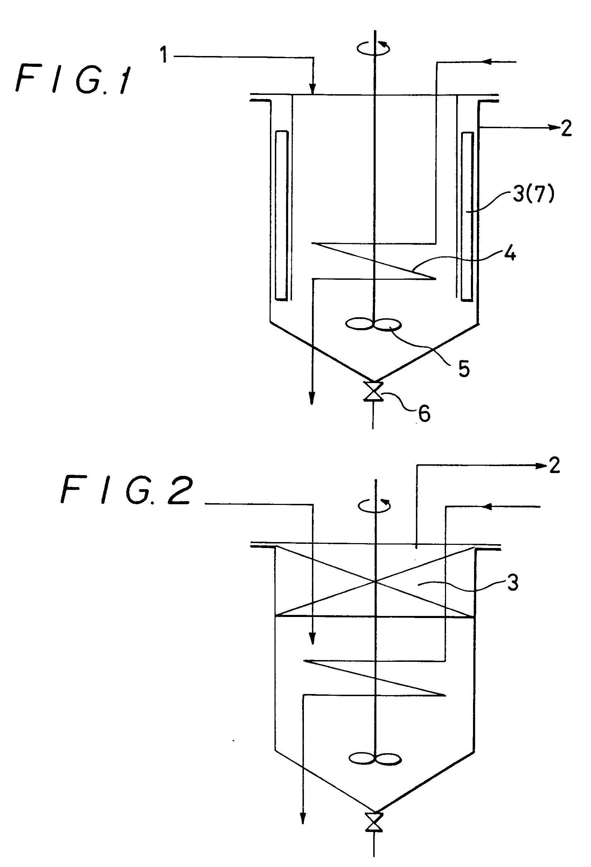

In the suspended bed process a stirred reactor is used. The main body of the reactor is provided with various auxiliary structures including a stirrer; catalyst charge and discharge ports; instruments such as a thermometer, a pressure gage, a level gage and nozzles for various operations. Moreover, the reactor generally has a number of accessory equipments disposed on the inside or outside thereof. Such equipments include a heat exchanger for controlling the reaction temperature; a settler for separating fine catalyst particles from the effluent reacted solution (for example, a settler as described in U.S. Patent 3,985,806 or as illustrated in Fig. 1 of the accompanying drawings); a filter.

The reaction is carried out in a continuous system. In this reaction, acrylonitrile and water can basically be used in any desired proportion. However, the weight ratio of acrylonitrile to water preferably ranges from 60:40 to 5:95 and more preferably from 50:50 to 10:90. The reaction temperature is preferably in the range of 70 to 150°C and more preferably in the range of 90 to 140°C. The degree of conversion of acrylonitrile to acrylamide is preferably in the range of 10 to 98% and more preferably in the range of 30 to 95%.

In the above-described reaction system, unreacted acrylonitrile, unreacted water and formed acrylamide may separate into two phases, instead of forming a homogeneous solution system. In such a case, acrylamide or other inert solvent may be added as a co-solvent. The pressure within the reactor is kept at the vapor pressure determined by the aforesaid temperature and composition, or at that vapor pressure plus the pressure of an inert gas such as nitrogen. Usually, the pressure ranges from atmospheric pressure to $2 \cdot 10^6$ Pa (20 atmospheres).

In the above-described reaction system, the selectivity of the reaction is very high. Usually, 98% or more of the reacted acrylonitrile is converted to acrylamide and the remainder is converted to acrylic acid or its salts, $\beta$-hydroxypropionitrile and $\beta$-hydroxypropionamide. As is well known, acrylonitrile, acrylamide and acrylic acid or its salts are polymerizable vinyl monomers and tend to polymerize under the above-described reaction conditions including a temperature of around 100°C.

Conventionally known stabilizers for preventing the polymerization of acrylonitrile includes ammonia, hydroquinone, hydroquinone monomethyl ether, copper and cuprous chloride-methylene blue mixtures. Similarly, copper powder, copper salts, iron salts, manganese salts, hydroquinone, thiourea, phenothiazine, cupferron and oxygen gas are known as stabilizers for acrylamide.

On the other side, the following two contrastive phenomena are known about the influence of oxygen and other oxidizing agents on the catalyst or the reaction system.

That is, U.S. Patent 3,642,894, G.B. 1,392,380 and DE-A-25 04 640 show that, when the catalyst is oxidized by oxygen, its activity is reduced and the amount of $\beta$-hydroxypropionitrile formed as a by-product is increased. Accordingly, it is regarded as effective to handle the catalyst so as not to bring it into contact with air, to previously remove any dissolved oxygen from the raw materials (i.e., acrylonitrile and water), and to remove oxygen from the gaseous phase if it is present at the upper part of the reactor. Moreover, where the catalyst is used as a suspended bed, it is necessary to separate it from the reacted solution by settling or filtration. However, if the catalyst is deteriorated by contact with oxygen, this settling or filtration may become difficult.

In contrast, it is described in the Journal of Japanese Chemical Society, 1981, No. 11, 1825 that the use of raw materials containing dissolved oxygen causes an enhancement in catalytic activity until a certain limit is reached. It is also described in E.P. 78,178 and D.O.S. 2,001,903 that partial oxidation of the catalyst rather enhances its activity. Moreover, some processes are known in which a copper salt (U.S. Patent 3,911,009) or a nitrate (U.S. Patent 4,169,107) is added to the reaction system. These additives are thought to be effective in oxidizing the catalyst partially. In fact, it is shown in the aforesaid E.P. 78,178 that, if the catalyst is previously treated with oxygen or a chlorate so that it is partially oxidized in an amount of 0.1 to 30% by weight, preferably 0.5 to 20% by weight, and more preferably 1 to 15% by weight, its catalytic activity can be enhanced without increasing the amount of $\beta$-hydroxypropionitrile formed as a by-product.

In industrial production, it is often required to stop the operation and discharge the catalyst from the reactor after the reaction has been continued for a certain period of time. For example, it may be necessary to discard the catalyst deactivated as a result of long-term use and charge a fresh catalyst. Moreover, it is also a common practice to transfer the deactivated catalyst to another apparatus, regenerate it, for example, according to the method proposed in U.S. Patent 3,766,088, and recharge it into the reactor. Furthermore, internal inspection and reconstruction of a reactor also make it necessary to discharge its contents.

Conventionally, where a desired procedure is carried out after essentially all of the catalyst has been discharged, it has been regarded as unnecessary to give the special consideration described above to the reactor during shut-down. According to the present inventors' practical experience, it has been recognized that, since a reactor for use in a process utilizing a metallic copper-based catalyst as a suspended bed has various auxiliary structures and accessory equipments as described above, a small amount of catalyst tends to adhere to or deposit on these auxiliary structures and accessory equipments and thereby remain in the reactor, even if it is intended to discharge all of the catalyst. Actually, as will be illustrated by the Comparative Examples and Examples given later, it is impossible to discharge the catalyst completely by resorting to conventional washing or cleaning means. In such a case, the amount of the residual catalyst is small, i.e., at most 5% by weight based on the amount of catalyst used at the beginning of the operation. Accordingly, it has been thought in the past that, even if such a small amount of residual catalyst is deteriorated (for example, by contact with air) during shut-down of the reactor, the subsequent supply of a large amount of intact catalyst restricts the reduction in catalytic activity, the increase of by-products, and

4

the impairment of settleability and filterability to a negligible extent. In fact, this problem has never been taken up as a subject for discussion.

In contradiction to the above-described well-known facts, considerations and experience, the present inventors have now found that, if a reactor containing a residual catalyst (though it is present in a small amount of, for example, 5% or less) is exposed to an atmosphere of air during shut-down of the reactor, the following serious characteristic phenomena will occur after the resumption of operation.

(1) The activity of the catalyst is normal immediately after resumption, but subsequently shows a rapid reduction.

(2) The settleability and filterability of the catalyst are normal immediately after resumption. With the lapse of time, however, the catalyst loses its settleability rapidly and the filter becomes clogged prematurely.

(3) Polymers are formed within the reactor.

On the basis of these unexpected findings, the present inventors have now devised a method for dealing with a small amount of catalyst remaining in such a reactor.

The present invention is directed to a method for stopping and starting a reactor after the reaction has been continued for a period of time, for example, until the efficient catalytic action cannot be maintained any longer. This method is effectively used for total or partial shut-down of a single-vessel or multi-vessel stirred reactor having a heat exchanger, a settler and a filter and the like as described above.

The method of the present invention dictates discharging the metallic copper-based catalyst, or discharging the catalyst and maintaining the reactor, in such a way that the amount of copper oxides present in the metallic copper-based catalyst remaining in the reactor, as expressed in terms of metallic copper, is not greater than 0.07% by weight based on the amount of metallic copper catalyst used at the beginning of the operation. However, as described above, a suspended bed reactor in which the reaction is carried out in the presence of a metallic copper-based catalyst used as a suspended bed has a complex equipment structure and, therefore, the copper oxide level of not greater than 0.07% by weight cannot be achieved by resorting to conventional means for discharging the catalyst and maintaining the reactor.

In the method of the present invention, the catalyst is discharged, or the catalyst is discharged and the reactor is maintained, in such a way that the amount of copper oxides present in the catalyst remaining in the reactor, as expressed in terms of metallic copper (the same shall apply hereinafter), is not greater than 0.07% by weight, and more preferably not greater than 0.03% by weight. Specifically, the method of the present invention involves two technical ideas.

That is, one of them comprises positively discharging and washing off the catalyst until the total amount of metallic copper and copper oxides remaining in the reactor is not greater than 0.07% by weight, whereby the above-described difficulties are overcome even if the reactor is exposed to an oxydizing atmosphere. The other comprises preventing the interior of the reactor from being exposed to an oxidizing atmosphere, whereby the same effect can be achieved even if the amount of metallic copper remaining in the reactor exceeds 0.07% by weight based on the amount of metallic copper catalyst used at the beginning of the operation.

More specifically, the above-described first technical idea can be embodied by discharging most of the catalyst from the reactor and then subjecting the reactor to positive washing (for example, washing with pressurized water spray), dissolution in dilute nitric acid and the like, or mechanical means such as cleaning and wiping. On the other hand, the second technical idea can be embodied by filling the reactor with water or preferably with deoxygenated water; filling the reactor with an inert gas (such as nitrogen, water vapor and helium) which does not have reactivity with the metallic copper-based catalyst; or reducing the partial pressure of oxygen within the reactor to a value of $6.66 \cdot 10^3$ Pa (50 torr) or less, and more preferably a value of $1.33 \cdot 10^3$ Pa (10 torr) or less, for example, by evacuating the reactor. These specific means may be suitably modified and used on the basis of the above-described two technical ideas.

The present invention is further illustrated by the following Examples and Comparative Examples.

Comparative Example 1

Preparation of catalyst:

A Raney copper alloy consisting of aluminum and copper in a weight ratio of 50:50 was powdered and sieved to remove coarse particles having a size of 80 mesh or greater. This alloy powder was added to a 25% aqueous solution of sodium hydroxide kept at 50 - 60°C over a period of 2 hours and then maintained at 50 - 60°C for an additional hour to form a so-called Raney copper catalyst. This catalyst was thoroughly washed with water by decantation and stored in water.

Reaction:

A 10-liter reactor made of stainless steel and equipped with a stirrer, a built-in heating steam coil and a built-in catalyst filter was used. First of all, the internal space of the reactor was filled with nitrogen and charged with 2 kg of the aforesaid catalyst as immersed in water. Then, acrylonitrile and water through which nitrogen gas had been blown to remove any dissolved oxygen were fed thereto at rates of 3 kg/hr and 6 kg/hr, respectively. Thus, using the steam coil to maintain a temperature of 120°C, the reaction was continued for about 3 weeks with stirring.

Results of reaction:

The reaction solution obtained through the catalyst filter was collected and analyzed. The analytical results are shown in Table 1.

## Table 1

| Day of sample collection | Degree of conversion of AN (%) | Amount of HPN formed as a by-product (% of AAM) |
|---|---|---|
| Day 1 | 69 | 0.08 |
| Day 6 | 66 | 0.08 |
| Day 11 | 65 | 0.10 |
| Day 16 | 63 | 0.12 |
| Day 21 | 61 | 0.12 |

(Note)  AN represents acrylonitrile, HPN represents β-hydroxypropionitrile, and AAM represents acrylamide.  (The same shall apply hereinafter.)

Discharge of catalyst:

After about 3 weeks, the feed of acrylonitrile was discontinued, the temperature of the reactor was reduced, and its washing with water by feeding water to the reactor was continued. Then, the feed of water was also discontinued. While the stirring was being continued, the catalyst was discharged through the catalyst discharge valve provided at the bottom of the reactor. As a result of this procedure, the internal space of the reactor was filled with air and the reactor was allowed to stand in that state for a week. Another experiment showed that about 20 g of the catalyst remained in the reactor after the catalyst was withdrawn according to the above-described procedure and that about 12 g of the residual catalyst would be oxidized by allowing it to stand in an atmosphere of air for a week.

Resumption of reaction:

After a week, the reactor was charged with another batch of unused catalyst which had previously been prepared and stored, and the reaction was resumed using the same procedure and conditions as described previously. During the period of 4 hours after resumption, the reaction seemed to be normal. After 24 hours, however, the flow resistance of the catalyst filter increased from the normal value of 0.1 kg/cm$^2$ or less to

$0.5$ kg/cm$^2$, suggesting that the filter was clogged. After 48 hours, the flow resistance further increased to 2.6 kg/cm$^2$, so that it was judged difficult to continue the experiment. Thus, the whole system was shut down according to the same procedure as described previously and it was tried to withdraw the catalyst from the bottom of the reactor. However, its withdrawal was immpossible owing to clogging of the pipe. In this experiment, the amount of copper oxides present in the catalyst used at the beginning of the operation was 0.6% by weight.

Results of resumed reaction:

As can be seen from the data of Table 2, it was found that the activity of the catalyst was reduced rapidly.

Table 2

| Time of sample collection (after re-sumption) | Degree of conversion of AN (%) | Amount of HPN formed as a by-product (% of AAM) | Flow resistance of filter (kg/cm$^2$) |
|---|---|---|---|
| 4 hours | 60 | 0.12 | 0.1 or less |
| 24 hours | - | - | 0.5 |
| 48 hours | 26 | 0.30 | 2.6 |

Inspection of reactor and catalyst:

When the reactor was uncovered and inspected, a large amount of polymer was found to be mixed in the catalyst. Moreover, it was observed that a large amount of polymer was sticking to the steam coil and the catalyst filter.

Example 1A

Reaction was carried out for about 3 weeks in the same manner as described in Comparative Example 1. Then, the catalyst was discharged, when nitrogen was introduced into the reactor in such a manner as to prevent air from entering the reactor. Thus, the reactor was maintained under a nitrogen pressure of $2 \cdot 10^5$ Pa G (2 kg/cm$^2$G) for a week. Another experiment showed that, if the reactor was maintained in that state, only 1 g or less of the catalyst remaining therein would be oxidized. After a week, the reactor was charged with 2 kg of unused catalyst, and the reaction was resumed and continued for about 3 weeks in the same manner as described in Comparative Example 1. In this experiment, the amount of copper oxides was 0.05% by weight.

7

The results of the reaction carried out during the aforesaid two periods are shown in Table 3.

### Table 3

| Day of sample collection | Degree of conversion of AN (%) | Amount of HPN formed as a by-product (% of AAM) |
|---|---|---|
| Day 1 | 68 | 0.08 |
| Day 6 | 65 | 0.09 |
| Day 11 | 64 | 0.09 |
| Day 16 | 62 | 0.11 |
| Day 21 | 62 | 0.12 |
| (after resumption) | | |
| Day 1 | 70 | 0.07 |
| Day 6 | 67 | 0.08 |
| Day 11 | 66 | 0.09 |

### Table 3 (cont'd)

| Day 16 | 64 | 0.11 |
|---|---|---|
| Day 21 | 63 | 0.11 |

Throughout the experimental period, the flow resistance of the catalyst filter remained at 0.1 kg/cm$^2$ or less. After completion of the experiment, the reactor was washed with water in the same manner as described in Comparative Example 1. Then, the catalyst was withdrawn, immersed in water and stored under an atmosphere of nitrogen. No polymer was mixed in this catalyst. When the reactor was uncovered and its interior was inspected, no polymer was found.

Example 1B

After completion of Example 1A, most of the catalyst remaining in the reactor was carefully washed off by exposure to pressurized water spray. Another experiment showed that, according to this procedure, the amount of catalyst remaining in the reactor was about 0.2 g and all of it would be oxidized by allowing the reactor to stand in air for a week. After this reactor was allowed to stand in an atmosphere of air for about 1 week, the reactor was charged with the catalyst which had been recovered in Example 1A and stored, and the reaction was resumed in the same manner as described in Example 1A. The results obtained by continuing this reaction for about 3 weeks are shown in Table 4.

## Table 4

| Day of sample collection (after re-sumption) | Degree of con-version of AN (%) | Amount of HPN formed as a by-product (% of AAM) |
|---|---|---|
| Day 1 | 63 | 0.12 |
| Day 6 | 61 | 0.14 |
| Day 11 | 60 | 0.14 |
| Day 16 | 58 | 0.15 |
| Day 21 | 57 | 0.15 |

Throughout the experimental period, the flow resistance of the catalyst filter remained at 0.1 kg/cm$^2$ or less. When the interior of the reactor was inspected after completion of the experiment, no polymer was found. In this experiment, the amount of copper oxides was 0.01% by weight.

Comparative Example 2

Catalyst:

A catalyst prepared in the same manner as described in Comparative Example 1 was used.

Reactor: (See Fig. 1.)

A 10-liter reactor made of stainless steel and having disposed therein a catalyst settler section 3, a heating steam coil 4 and a stirrer 5 was used. This reactor comprises a vertically positioned cylindrical main body having a diameter of 220 mm, a conical bottom, and a top cover consisting of a flat plate. The settler section is the space formed between the main body and an inner tube of 170 mm diameter disposed therein. In this space, 12 slide-like slanting plates 7 are disposed at regular intervals. The raw materials are fed through a raw material feed pipe 1 to the upper part of the reactor and reacted within the inner tube. The reacted solution goes up obliquely along the slanting plates of the settler section and leaves the reactor through a reacted solution discharge pipe 2. In order to withdraw the catalyst after completion of an experiment, a catalyst discharge valve 6 is provided at the bottom.

Reaction:

Reaction was carried out for about 1 week under the same conditions as described in Comparative Example 1. The results thus obtained are shown in Table 5.

Table 5

| Day of sample collection | Degree of conversion of AN (%) | Amount of HPN formed as a by-product (% of AAM) |
|---|---|---|
| Day 1 | 69 | 0.08 |
| Day 3 | 65 | 0.08 |
| Day 5 | 64 | 0.09 |
| Day 7 | 64 | 0.10 |

Discharge of catalyst:

After about 1 week, the feed of acrylonitrile was discontinued, the temperature of the reactor was reduced, and its washing with water was continued. Then, the feed of water was also discontinued. While the stirring was being continued, the catalyst was discharged through the catalyst discharge valve into a vessel. This catalyst was stored in water and replacing the gaseous phase of the vessel with nitrogen. Further, it was tried to wash off the catalyst remaining in the reactor by exposure to pressurized water spray. Another experiment showed that about 100 g of the catalyst remained in the reactor (mainly on the slanting plates of the settler section) after the catalyst was withdrawn according to the above-described procedure and that about 40 g of the residual catalyst would be oxidized by allowing it to stand in an atmosphere of air for a week.

Resumption of reaction:

After the reactor was allowed to stand in an atmosphere of air for a week, it was charged again with the catalyst, which has been discharged and stored, handling so as not to bring it into contact with air, and the reaction was resumed under the same conditions as described previously. On the day of resumption, the reaction seemed to be normal. After 12 hours, however, a sample of the reacted solution separated into two layers when cooled, suggesting that the degree of conversion was reduced to 50% or less. After 16 hours, not only the reacted solution separated into two layers more markedly, but also a large number of polymer particles were found to be mixed in the reacted solution. Thus, the whole system was shut down according to the same procedure as described previously.

Inspection of reactor and catalyst:

When the reactor was uncovered and inspected, a large amount of polymer was found to be mixed in the catalyst. Moreover, it was noted that a large amount of polymer, together with a small amount of the catalyst, was sticking to the slanting plates of the settler section. In this experiment, the amount of copper oxides was 2% by weight.

Example 2

Reaction was carried out for a week in the same manner as described in Comparative Example 2. Then, the catalyst was withdrawn, immersed in water and stored under an atmosphere of nitrogen. The reactor was immediately filled with water and allowed to stand for a week. Another experiment showed that the amount of the catalyst oxidized under these conditions would be 1 g or less. After a week, the reactor was charged with the stored catalyst, and the reaction was resumed and continued for about 1 week in the same manner as described in Comparative Example 2.

The results of the reaction carried out during the aforesaid two periods are shown in Table 6.

Table 6

| Day of sample collection | Degree of conversion of AN (%) | Amount of HPN formed as a by-product (% of AAM) |
|---|---|---|
| Day 1 | 68 | 0.08 |
| Day 3 | 66 | 0.08 |
| Day 5 | 66 | 0.09 |
| Day 7 | 64 | 0.09 |
| (after resumption) | | |
| Day 1 | 61 | 0.10 |
| Day 3 | 60 | 0.10 |
| Day 5 | 57 | 0.10 |
| Day 7 | 57 | 0.12 |

After completion of the aforesaid reaction, the whole system was shut down in the same manner as described in Comparative Example 2. When the reactor was uncovered and inspected, no polymer was found. In this experiment, the amount of copper oxides was 0.05% by weight.

Comparative Examples 3

Catalyst:

A catalyst prepared in the same manner as described in Comparative Example 1 was used.

Reactor: (See Fig. 2.)

A 10-liter reactor made of stainless steel and having disposed therein a catalyst settler section 3, a heating steam coil and a stirrer was used. This reactor comprises a vertically positioned cyclindrical main body having a diameter of 220 mm, a conical bottom, and a top cover consisting of a flat plate. In the upper part of the reactor, 24 wire nets having an opening size of 3 mm x 3 mm are laid horizontally one on top of another so as to form a settler section. The raw materials are fed through a raw material feed pipe 1 to the interior of the reactor and reacted in the lower part of the reactor. The reacted solution goes up through the settler section and leaves the reactor through a reacted solution discharge pipe 2. In order to withdraw the catalyst after completion of an experiment, a catalyst discharge valve is provided at the bottom.

Reaction:

Reaction was carried out for about 1 week under the same conditions as described in Comparative Example 1. The results thus obtained are shown in Table 7.

11

## Table 7

| Day of sample collection | Degree of conversion of AN (%) | Amount of HPN formed as a by-product (% of AAM) |
|---|---|---|
| Day 1 | 70 | 0.07 |
| Day 3 | 67 | 0.08 |
| Day 5 | 64 | 0.09 |
| Day 7 | 63 | 0.09 |

Discharge of catalyst:

After a week, the feed of acrylonitrile was discontinued, the temperature of the reactor was reduced, and its washing with water was continued. Then, the feed of water was also discontinued. While the stirring was being continued, the catalyst was discharged through the catalyst discharge valve into a vessel. Further, in order to recover the small amount of catalyst remaining in the settler section, the reactor was filled again with water and drained into the same vessel. The catalyst recovered in this manner was stored in water and replacing the gaseous phase of the vessel with nitrogen. Another experiment showed that about 50 g of the catalyst remained in the reactor (depositing mainly in the settler section) after the catalyst was withdrawn according to the above-described procedure and that about 40 g of the residual catalyst would be oxidized by allowing it to stand in an atmosphere of air for a week.

Resumption of reaction:

After the reactor was allowed to stand in an atmosphere of air for a week, it was charged again with the catalyst, which has been discharged and stored, handling so as not to bring it into contact with air, and the reaction was resumed under the same conditions as described previously. At the beginning, the reaction seemed to be normal. After 12 hours, however, a sample of the reacted solution separated into two layers when cooled, suggesting that the degree of conversion was reduced to 50% or less. Moreover, polymer particles were found to be present in the reacted solution. After 24 hours, the separation of the reacted solution into two layers and the presence of polymer particles therein became more marked. Thus, the whole system was shut down according to the same procedure as described previously.

Inspection of reactor and catalyst:

When the reactor was uncovered and inspected, a large amount of polymer was found to be mixed in the catalyst. Moreover, it was noted that a large amount of polymer, together with a small amount of the catalyst, was sticking to the wire nets of the settler section. In this experiment, the amount of copper oxides was 2% by weight.

Example 3

Reaction was carried out for a week in the same manner as described in Comparative Example 3. Then, the catalyst was withdrawn. Further, in order to recover the catalyst depositing in the settler section, the reactor was filled again with water and drained. As described in Comparative Example 3, the catalyst recovered in this manner was immersed in water and stored under an atmosphere of nitrogen. Then, the residual catalyst was dissolved out by filling the reactor with 10% nitric acid, stirring it for about 1 hour, and then washing the reactor with water. When the reactor was uncovered and inspected, it was confirmed that no catalyst remained therein. After a week, the reactor was charged again with the stored catalyst, and the reaction was resumed and continued for about 1 week in the same manner as described in Comparative Example 3.

The results of the reaction carried out during the aforesaid two periods are shown in Table 8.

## Table 8

| Day of sample collection | Degree of conversion of AN (%) | Amount of HPN formed as a by-product (% of AAM) |
|---|---|---|
| Day 1 | 70 | 0.07 |
| Day 3 | 66 | 0.08 |
| Day 5 | 63 | 0.08 |
| Day 7 | 61 | 0.10 |
| (after resumption) | | |
| Day 1 | 60 | 0.10 |
| Day 3 | 57 | 0.10 |
| Day 5 | 57 | 0.12 |
| Day 7 | 55 | 0.12 |

After completion of the aforesaid reaction, the whole system was shut down in the same manner as described in Comparative Example 3. When the reactor was uncovered and inspected, no polymer was found. In this experiment, the amount of copper oxides was 0% by weight.

Comparative Example 4

Preparation of catalyst:

A copper oxide-chromium oxide composition was prepared according to the procedure described in Example 1 of U.S. Patent 3,631,104. Specifically, to 25 parts (by weight) of ammonium dichromate dissolved in 100 parts of water, 30 parts of ammonium hydroxide was added to obtain ammonium chromate. To this ammonium chromate solution, a solution of 20.2 parts of cupric chloride dissolved in 150 parts of water was added slowly with continuous agitation. The resulting precipitate was separated and washed several timses with approximately one liter of water each time. The separated precipitate was dried at 100°C for 8 hours and then heated at 275°C in air for 3 hours. Using a tableting machine, the resulting composition was formed into cylindrical tablets having a diameter of 5 mm and a length of 5 mm. A stainless steel reaction tube having an internal diameter of 50 mm was charged with 2000 g (1600 g as copper) of the tablets so formed. By passing hydrogen gas diluted with nitrogen through this tube, the composition was reduced at 250°C for 4 hours to obtain the so-called reduced copper-chromium oxide catalyst. Then, this catalyst was immersed in water and ground under an atmosphere of nitrogen to a particle size of about 1 mm or less.

Reaction:

The internal space of the same reactor as used in Comparative Example 1 was first filled with nitrogen and then charged with the aforesaid catalyst as immersed in water. To this reactor, acrylonitrile and water through which nitrogen gas had previously been blown to remove any dissolved oxygen were fed at rates of 1.5 kg/hr and 3 kg/hr, respectively. Thus, stirring and using the steam coil to maintain a reaction temperature of 120°C, the reaction was continued for about 1 week. The results of the reaction carried out in this manner are shown in Table 9.

## Table 9

| Day of sample collection | Degree of conversion of AN (%) | Amount of HPN formed as a by-product (% of AAM) |
|---|---|---|
| Day 1 | 65 | 0.09 |
| Day 3 | 63 | 0.10 |
| Day 5 | 61 | 0.10 |
| Day 7 | 61 | 0.11 |

Discharge of catalyst:

After completion of the reaction experiment, the feed of acrylonitrile was discontinued, the temperature of the reactor was reduced, and its washing with water was continued. Then, the feed of water was also discontinued. While the stirring was being continued, the catalyst, together with the water, was discharged into a vessel through the catalyst discharge valve provided at the bottom of the reactor. In order to further recover the catalyst remaining in the reactor, the reactor was filled with water, followed by stirring and discharging the water. After the catalyst was withdrawn in this manner, it was observed that about 20 g of the catalyst was still deposited on or sticking to the inside surfaces of the reactor. Then, the reactor was allowed to stand under an atmosphere of air for about 1 week. Another experiment showed that most of the copper present in the residual catalyst would be oxidized under these conditions. The catalyst withdrawn in the above-described manner was stored in water and replacing the gaseous phase of the vessel with nitrogen gas.

Resumption of reaction:

After a week, the reactor was charged again with the catalyst, which had been discharged and stored, handling so as not to bring it into contact with air, and the reaction was resumed under the same conditions as described previously. On the day of resumption, the reaction seemed to be normal. After 24 hours, however, the flow resistance of the catalyst filter increased from the normal value of 0.1 kg/cm$^2$ or less to 0.2 kg/cm$^2$. After 48 hours, the flow resistance further increased to 1.2 kg/cm$^2$. Thus, the whole system was shut down according to the same procedure as described previously.

Results of resumed reaction:

As can be seen from the data of Table 10, it was found that the activity of the catalyst was reduced rapidly.

14

## Table 10

| Time of sample collection (after re-sumption) | Degree of conversion of AN (%) | Amount of HPN formed as a by-product (% of AAM) |
|---|---|---|
| 4 hours | 59 | 0.11 |
| 48 hours | 31 | 0.28 |

Inspection of reactor and catalyst:

When the reactor was uncovered and inspected, a large amount of polymer was found to be mixed in the catalyst, or the catalyst particles were found to be covered with a gel-like polymer. In this experiment, the amount of copper oxides was 1.25% by weight.

Example 4

Reaction was carried out for about 1 week in the same manner as described in the former stage of Comparative Example 4. Then, the catalyst was discharged. Immediately after that, a vacuum pump was connected to the reactor and the pressure within the reactor was kept at $1.33 \cdot 10^3$ Pa (10 torr) or less. In that state, the reactor was allowed to stand for a week. Another experiment showed that the amount of copper oxidized during this period was 1 g or less. Thereafter, the reaction was resumed and continued for about 1 week in the same manner as described in Comparative Example 4.

The results of the reaction carried out during the aforesaid two periods are shown in Table 11.

## Table 11

| Day of sample collection | Degree of conversion of AN (%) | Amount of HPN formed as a by-product (% of AAM) |
|---|---|---|
| Day 1 | 63 | 0.09 |
| Day 3 | 61 | 0.10 |
| Day 5 | 59 | 0.11 |
| Day 7 | 59 | 0.11 |
| (after re-sumption) | | |
| Day 1 | 58 | 0.13 |

15

Table 11 (cont'd)

| Day 3 | 56 | 0.13 |
|-------|----|------|
| Day 5 | 56 | 0.13 |
| Day 7 | 55 | 0.14 |

No polymer particles were observed in the reaction solution. Moreover, when the interior of the reactor was inspected after completion of the experiment, no polymer was found. In this experiment, the amount of copper oxides was 0.06% by weight.

## Claims

1. A method for stopping and starting an acrylamide reactor for use in a process of the synthesis of acrylamide by reacting acrylonitrile with water in the presence of a metallic copper-base catalyst used as a suspensed bed, stopping said reactor after a continous reaction, shutting the reactor down and discharging the catalyst, and thereafter restarting said reactor for repeating the reaction, said reactor being a stirred reactor having at least a heat exchanger, a catalyst settler or a catalyst filter disposed on the inside or outside thereof, characterized in that the following steps are carried out:

a) discharging the catalyst by the procedure of washing or dissolving at least a part of the metallic copper and/or copper oxides and washing off the resulting solution, whereby the total amount of metallic copper and copper oxides remaining in the reactor is decreased to not greater than 0.07% by weight based on the amount of metallic copper catalyst used at the beginning of the operation, or

b) discharging the catalyst, and then the reactor is either filled with water or inert gas or evacuated so that the partial pressure of oxygen within the reactor is kept at $6.66 \cdot 10^3$ Pa (50 torr) or less, whereby the amount of copper oxides remaining in the reactor prior to restart is decreased to not greater than 0.07% by weight based on the amount of metallic copper catalyst used at the beginning of the operation.

2. A method for stopping and starting an acrylamide reactor as claimed in claim 1, wherein the procedure for discharging the catalyst comprises dissolving at least a part of the metallic copper and/or copper oxides and washing off the resulting solution.

3. A method for stopping and starting an acrylamide reactor as claimed in claim 1, wherein after most of the catalyst has been discharged the partial pressure of oxygen within the reactor is kept at $6.66 \cdot 10^3$ Pa (50 torr) or less.

## Patentansprüche

1. Verfahren für den Abbruch und den Start eines Acrylamidreaktors zur Verwendung bei einem Verfahren zur Synthese von Acrylamid durch Umsetzung von Acrylnitril mit Wasser in Gegenwart eines als suspendiertes Bett verwendeten Katalysators auf Basis von metallischem Kupfer, Abbruch des Reaktors nach einer kontinuierlichen Reaktion, Abschalten des Reaktors und Entnahme des Katalysators, und hiernach erneutes Starten des Reaktors für die Wiederholung der Reaktion, wobei der Reaktor ein gerührter Reaktor mit zumindest einem Wärmeaustauscher, einem Katalysator-Absetzer oder einem Katalysatorfilter, angebracht an der Innen- oder Außenseite hiervon, ist, dadurch gekennzeichnet, daß die folgenden Stufen durchgeführt werden:

a) Entnahme des Katalysators durch das Verfahren eines Waschens oder Lösens zumindest eines Teils des metallischen Kupfers und/oder der Kupferoxide und Abwaschen der entstandenen Lösung, wodurch die in dem Reaktor verbliebene Gesamtmenge an metallischem Kupfer und Kupferoxiden auf nicht mehr als 0,07 Gew.-%, basierend auf der Menge des zu Beginn des Verfahrens verwendeten metallischen Kupferkatalysators, herabgesetzt wird, oder

b) Entnahme des Katalysators, wonach der Reaktor entweder mit Wasser oder einem Inertgas gefüllt oder derart evakuiert wird, daß der Partialdruck des Sauerstoffs in dem Reaktor bei $6{,}66.10^3$ Pa (50 Torr) oder weniger gehalten wird, wodurch die Menge der in dem Reaktor vor dem erneuten Start verbliebenen Oxide auf nicht mehr als 0,07 Gew.-%, basierend auf der Menge des zu Beginn des Verfahrens verwendeten metallischen Kupferkatalysators, herabgesetzt wird.

2. Verfahren für den Abbruch und den Start eines Acrylamidreaktors gemäß Anspruch 1, worin das Verfahren der Entnahme des Katalysators das Lösen zumindest eines Teils des metallischen Kupfers und/oder der Kupferoxide und das Abwaschen der entstandenen Lösung umfaßt.

3. Verfahren für den Abbruch und den Start eines Acrylamidreaktors gemäß Anspruch 1, worin nach Entnahme des überwiegenden Teils des Katalysators der Partialdruck des Sauerstoffs in dem Reaktor bei $6{,}66.10^3$ Pa (50 Torr) oder geringer gehalten wird.

**Revendications**

1. Procédé pour mettre en marche et arrêter un réacteur d'acrylamide destiné à l'utilisation dans un procédé de synthèse d'acrylamide en mettant en réaction de l'acrylonitrile avec de l'eau en présence d'un catalyseur métallique cuivre-base utilisé sous forme de lit en suspension, arrêter le réacteur après une réaction continue, fermer le réacteur et décharger le catalyseur, puis remettre le réacteur en route pour répéter la réaction, ce réacteur étant un réacteur à agitation avec au moins un échangeur de chaleur, un décanteur de catalyseur ou un filtre de catalyseur disposés à l'intérieur ou à l'extérieur de celui-ci, caractérisé en ce que l'on effectue les étapes suivantes consistant à :
   a) décharger le catalyseur par le procédé de lavage ou de dissolution d'au moins une partie des oxydes de cuivre métallique et/ou de cuivre et lavage pour éliminer la solution résultante, permettant de faire diminuer la quantité totale de cuivre métallique et d'oxydes de cuivre restant dans le réacteur à une valeur non supérieure a 0.07 % en poids rapportée à la quantité du catalyseur de cuivre métallique utilisée au début de l'opération ou
   b) décharger le catalyseur, puis charger le réacteur soit avec de l'eau du gaz inerte ou mettre sous vide de façon à ce que la pression partielle d'oxygène à l'intérieur du réacteur soit maintenue à $6{,}66.10^3$ Pa (50 torr) ou moins, permettant de diminuer la quantité d'oxydes de cuivre restant dans le réacteur avant le redémarrage à une valeur non supérieure à 0.07% en poids rapportêe à la quantité de catalyseur de cuivre métallique utilisé au début de l'opération.

2. Procédé pour mettre en marche et arrêter un réacteur d'acrylamide selon la revendication 1, dans lequel le procédé de décharge du catalyseur comprend la dissolution d'au moins une partie du cuivre métallique et/ou des oxydes de cuivre et l'élimination par lavage de la solution résultante.

3. Procédé pour mettre en marche et arrêter un réacteur d'acrylamide selon la revendication 1, dans lequel après avoir déchargé la plus grande partie du catalyseur la pression partielle d'oxygène à l'intérieur du réacteur est maintenue à $6{.}66.10^3$ Pa (50 torr) ou moins.

F I G.1

F I G.2